# EUROPÄISCHE PATENTSCHRIFT

(11) **EP 3 269 425 B1**
(45) Veröffentlichungstag und Bekanntmachung des Hinweises auf die Patenterteilung: **27.02.2019**
(21) Anmeldenummer: 17171694.7
(22) Anmeldetag: 18.05.2017
(51) Int. Cl.: A61Q 17/04, A61K 8/81

(54) **NEUSTES SONNENSCHUTZMITTEL MIT REDUZIERTER NEIGUNG ZUR TEXTILVERFLECKUNG**
NOVEL SUNSCREEN COMPOSITION WITH A REDUCED LIKELIHOOD TO DISCOLOUR TEXTILES
NOUVELLE PROTECTION SOLAIRE AYANT UNE TENDANCE RÉDUITE À TÂCHER LES TISSUS

(30) Priorität: 23.06.2016 DE 102016211239
(43) Veröffentlichungstag der Anmeldung: 17.01.2018
(73) Patentinhaber: Beiersdorf AG, 20253 Hamburg (DE)
(72) Erfinder: Weinert, Katrin, 22763 Hamburg (DE); Toshihiko, Shimoda, 20257 Hamburg (DE); Bleckmann, Andreas, 22926 Ahrensburg (DE)

(56) Entgegenhaltungen:
- EP-A1- 2 939 710
- EP-A1- 3 144 035
- EP-A1- 3 150 190
- WO-A2-2014/102050

## Beschreibung

Die vorliegende Erfindung betrifft Verfahren und Verwendungen zur Erleichterung der Auswaschbarkeit von UV-Lichtschutzfilter enthaltenden kosmetischen Zubereitungen aus Textilien, dadurch gekennzeichnet, dass dem Kosmetikum Polyvinylalkohol zugesetzt wird.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und Wege zu finden, um eine kosmetische Zubereitung (insbesondere ein Sonnenschutzmittel) enthaltend nicht-wasserlösliche UV-A-Filter und/oder Breitbandfilter leichter aus den mit der Zubereitung kontaminierten Textilen herauswaschen zu können.

Überraschend gelöst wird die Aufgabe durch ein Verfahren zur Erleichterung der Auswaschbarkeit von UV-Lichtschutzfilter enthaltenden kosmetischen Zubereitungen aus Textilien, dadurch gekennzeichnet, dass dem Kosmetikum Polyvinylalkohol zugesetzt wird.

Überraschend gelöst wird die Aufgabe durch ein Verfahren zur Reduzierung der durch UV-Lichtschutzfilter enthaltenden kosmetischen Zubereitungen hervorgerufenen Textilverfleckung, dadurch gekennzeichnet, dass dem Kosmetikum Polyvinylalkohol zugesetzt wird.

Überraschend gelöst wird die Aufgabe durch die Verwendung von Polyvinylalkohol in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

Überraschend gelöst wird die Aufgabe durch die Verwendung von Polyvinylalkohol in UV-Lichtschutzfilter enthaltenden kosmetischen Zubereitungen zur Reduzierung der durch die Zubereitung hervorgerufenen Textilverfleckung.

Zwar wurde in der Fachwelt der Einsatz von Polyvinylalkohol in Sonnenschutzmitteln immer wieder mal erwogen. So offenbaren die DE 102005059742, WO 2014/102050 oder die US 2004/0126339, die Möglichkeit, Polyvinylalkohol in Sonnenschutzmittel einzuarbeiten. Dennoch konnten diese Schriften nicht den Weg zur vorliegenden Erfindung weisen, da die Wirkung des Polyvinylalkohols auf die Auswaschbarkeit nicht erkannt wurde.

Erfindungsgemäß vorteilhafte Ausführungsformen der vorliegenden Erfindung sind dadurch gekennzeichnet, dass als Polyvinylalkohol einen Hydrolysegrad (Verseifungsgrad) von 86 bis 89 Mol-% aufweist. Dabei ist ein Hydrolysegrad (verseifungsgrad) von 86,7 bis 88,7 Mol-% erfindungsgemäß bevorzugt.

Darüber hinaus ist es erfindungsgemäß von Vorteil, wenn als Polyvinylalkohol eine Viskosität gemessen nach DIN 53015 (4%ige wässrige Lösung bei 20 °C) von 3 bis 50 mPas aufweist.

Die Begriffe "erfindungsgemäße Zubereitung", "erfindungsgemäß" etc. beziehen sich im Rahmen der vorliegenden Offenbarung immer auf das erfindungsgemäße Verfahren und die erfindungsgemäße Verwendung.

Die Begriffe kosmetische Zubereitung und Kosmetikum werden synonym verwendet.

Die erfindungsgemäßen Verfahren und Verwendungen sind erfindungsgemäß vorteilhaft dadurch gekennzeichnet, dass die kosmetische Zubereitung bzw. das Kosmetikum die Verbindungen 4-(tert.-Butyl)-4'-methoxydibenzoylmethan (INCI Butyl Methoxydibenzoylmethane) und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

Dabei sind die drei erfindungsgemäßen Varianten wie folgt gekennzeichnet:
Enthält die kosmetische Zubereitung 4-(tert.-Butyl)-4'-methoxydibenzoylmethan (INCI Butyl Methoxydibenzoylmethane) und kein Bis-Ethylhexyloxyphenol Methoxyphenyl Triazine, so ist es erfindungsgemäß vorteilhaft, wenn der Gehalt an 4-(tert.-Butyl)-4'-methoxydibenzoylmethan (INCI Butyl Methoxydibenzoylmethane) von 0,1 bis 5 Gewichts-%, bezogen auf das Gesamtgewicht der Zubereitung beträgt.

Enthält die kosmetische Zubereitung 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) und kein Butyl Methoxydibenzoylmethane, so ist es erfindungsgemäß vorteilhaft, wenn der Gehalt an 2,4-Bis-{[4-(2-ethyl-hexyloxy)-2-hydroxyl-phenyl}-6-(4-methoxyphenyl)-1,3,5-triazin (INCI Bis-Ethylhexyloxyphenol Methoxyphenyl Triazine) von 0,1 bis 10 Gewichts-%, bezogen auf das Gesamtgewicht der Zubereitung beträgt.

Enthält die kosmetische Zubereitung 4-(tert.-Butyl)-4'-methoxydibenzoylmethan (INCI Butyl Methoxydibenzoylmethane) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine), so ist es erfindungsgemäß vorteilhaft, wenn der Gehalt an 4-(tert.-Butyl)-4'-methoxydibenzoylmethan (INCI Butyl Methoxydibenzoylmethane) von 0,1 bis 5 Gewichts-% und der Gehalt an 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) von 0,1 bis 10 Gewichts-%, jeweils bezogen auf das Gesamtgewicht der Zubereitung beträgt.

Es ist für das erfindungsgemäße Verfahren und die erfindungsgemäße Verwendung erfindungsgemäß vorteilhaft, wenn die kosmetische Zubereitung bzw. das Kosmetikum von 0,01 bis 2,5 Gewichts-% Polyvinylalkohol, bezogen auf das Gesamtgewicht der Zubereitung, enthält.

Es ist für das erfindungsgemäße Verfahren und die erfindungsgemäße Verwendung erfindungsgemäß bevorzugt, wenn die kosmetische Zubereitung bzw. das Kosmetikum von 0,1 bis 1,5 Gewichts-% Polyvinylalkohol, bezogen auf das Gesamtgewicht der Zubereitung, enthält.

Darüber hinaus kann die kosmetische Zubereitung vorteilhaft noch weitere UV-Filter enthalten, die beispielsweise gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenyl-acrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate;Titandioxid; Zinkoxid.

Dabei ist es erfindungsgemäß von Vorteil, wenn die erfindungsgemäße Zubereitung frei ist von 2-Ethylhexyl-2-cyano-3,3-diphenylacrylat, 2-Hydroxy-4-methoxybenzophenon und/oder 3-(4-Methylbenzyliden)campher. Dabei sollte bevorzugt auf den Einsatz von 3-(4-Methylbenzyliden)campher und 2-Hydroxy-4-methoxybenzophenon verzichtet werden.

Der erfindungsgemäße Effekt kann dadurch erfindungsgemäß verbessert werden, dass man dem Kosmetikum ein oder mehrere Komplexbildner zusetzt.

Dabei ist es erfindungsgemäß bevorzugt, wenn als Komplexbildner eine oder mehrere der Verbindungen aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat (IDS)
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze eingesetzt werden.

Es ist erfindungsgemäß besonders bevorzugt, wenn als Komplexbildner eine oder mehrere der Verbindungen aus der Gruppe
- Diethylentriaminpenta(methylenphosphonsäure)/ DTPMP
   - Ethylendiamintetra(methylenphosphonsäure/ EDTMP
   - Aminotrimethylenphosphonsäure/ ATMP
   - Phosphonobutan-tricarbonsäure/ PBTC
   - Iminodisuccinat (IDS)
   - Natriumpolyphosphat
   - Tetranatriumpyrophosphat
   - Bernsteinsäure
   und/oder deren Alkalisalze und/ oder deren Amin-N-Oxide eingesetzt werden.

Die erfindungsgemäß bevorzugte Gesamt-Einsatzkonzentration für die Komplexbildner (einer oder mehrere) beträgt dabei von 0,01 bis 3 Gewichts-%, bezogen auf das Gesamtgewicht der Zubereitung.

Der erfindungsgemäße Effekt kann dadurch erfindungsgemäß verbessert werden, dass man dem Kosmetikum 1-Hydroxy-4-methyl-6-(2,4,4-trimethylpentyl)-2(1H)-pyridon (Pirocton) und/oder dessen Monoethanolamin-Salz zugesetzt wird.

Die erfindungsgemäß bevorzugte Gesamt-Einsatzkonzentration für Pirocton beträgt dabei von 0,01 bis 1 Gewichts-%, bezogen auf das Gesamtgewicht der Zubereitung.

Der erfindungsgemäße Effekt kann dadurch erfindungsgemäß verbessert werden, dass man dem Kosmetikum Siloxanelastomere zusetzt.

Die erfindungsgemäß bevorzugte Gesamt-Einsatzkonzentration für Siloxanelastomere (einer oder mehrere) beträgt dabei von 0,1 bis 20 Gewichts-%, bezogen auf das Gesamtgewicht der Zubereitung.

Nicht zuletzt kann der erfindungsgemäße Effekt dadurch erfindungsgemäß verbessert werden, dass man dem Kosmetikum ein oder mehrere Polysaccharide zusetzt.

Die erfindungsgemäßen Polysaccharide können aus unterschiedlichen Stoffgruppen gewählt werden.

So ist eine erfindungsgemäß vorteilhafte Ausführungsform der vorliegenden Erfindung dadurch gekennzeichnet, dass die verwendeten Polysaccharide ausgewählt werden aus der Gruppe der Gumen.

In einem solchen Fall ist es erfindungsgemäß bevorzugt, wenn die verwendeten Polysaccharide ausgewählt werden aus der Gruppe der Verbindungen Welan Gum, Sclerotium Gum und Cellulose Gum.

Die erfindungsgemäß vorteilhaften Polysaccharide können jedoch auch ausgewählt werden aus der Gruppe der Verbindungen Alginate (insbesondere Sodium Alginate) und Carboxymethylcellulose.

Es ist in jedem Falle erfindungsgemäß vorteilhaft, wenn die Zubereitung Polysaccharide in einer Gesamtmenge von 0,01 bis 2 Gewichts-%, bezogen auf das Gesamtgewicht der Zubereitung, enthält.

Für die erfindungsgemäß vorteilhaften Polysaccharide gelten für die einzelnen Stoffe die folgenden Einsatzkonzentrationen als erfindungsgemäß bevorzugt:
Welan Gum wird erfindungsgemäß bevorzugt in einer Konzentration von 0,01 bis 1,5 Gewichts-%, bezogen auf das Gesamtgewicht der Zusammensetzung eingesetzt. Als Welan Gum kann beispielsweise der Rohstoff Collstab W-100 der Firma Colltec vorteilhaft eingesetzt werden.

Sclerotium Gum wird erfindungsgemäß bevorzugt in einer Konzentration von 0,01 bis 1,5 Gewichts-%, bezogen auf das Gesamtgewicht der Zusammensetzung eingesetzt. Als Sclerotium Gum kann beispielsweise der Rohstoff Actigum CS 11der Firma Cargill vorteilhaft eingesetzt werden.

Cellulose Gum wird erfindungsgemäß bevorzugt in einer Konzentration von 0,01 bis 1,5 Gewichts-%, bezogen auf das Gesamtgewicht der Zusammensetzung eingesetzt. Als Cellulose Gum kann beispielsweise der Rohstoff Blanose Cellulose Gum der Firma Ashland vorteilhaft eingesetzt werden.

Sodium Alginate wird erfindungsgemäß bevorzugt in einer Konzentration von 0,01 bis 1,5 Gewichts-%, bezogen auf das Gesamtgewicht der Zusammensetzung eingesetzt. Als Sodium Alginate kann beispielsweise der Rohstoff Alginic Acid Sodium Salt der Firma Sigma Aldrich vorteilhaft eingesetzt werden.

Carboxymethylcellulose wird erfindungsgemäß bevorzugt in einer Konzentration von 0,01 bis 2 Gewichts-%, bezogen auf das Gesamtgewicht der Zusammensetzung eingesetzt. Als Carboxymethylcellulose kann beispielsweise der Rohstoff Aqualon CMC der Firma Ashland vorteilhaft eingesetzt werden.

Es ist erfindungsgemäß vorteilhaft, wenn man dem Kosmetikum 4-Hydroxyacetophenon zusetzt. Enthält das Kosmetikum 4-Hydroxyacetophenon, so beträgt die erfindungsgemäß vorteilhafte Einsatzkonzentration 0,01-2 Gewichts-%, bezogen auf das Gesamtgewicht des Kosmetikums.

Erfindungsgemäß vorteilhafte Ausführungsformen der vorliegenden Erfindung sind dadurch gekennzeichnet, dass die Zubereitung in Form einer Öl-in-Wasser-Emulsion (O/W-Emulsion) vorliegt.
In einem solchen Fall sind die erfindungsgemäß bevorzugten Ausführungsformen dadurch gekennzeichnet, dass die Zubereitung einen oder mehrere Emulgatoren gewählt aus der Gruppe der Verbindungen Glycerylstearatcitrat, Cetearylalkohol
Natriumcetearylsulfat + Glycerylstearat, Cetearylsulfosuccinat Natriumstearoylglutamat, Polyglyceryl-3-methylglucosedistearat,
Stearinsäure, Kaliumcetylphosphat, enthalten.

Darüber hinaus kann die kosmetische Zubereitung wie ein für solche Fälle übliches Kosmetikum zusammengesetzt sein und die entsprechenden, bekannten Inhaltsstoffe enthalten.

### Vergleichsversuch/Ausführungsbeispiel

### Vergleichsversuch

Mit dem folgenden Versuch konnte der erfindungsgemäße Effekt beispielhaft belegt werden:
Es wurden die erfindungsgemäßen Polyvinylalkohole zu einer Butyl Methoxydibenzoylmethane und Bis-Ethylhexyloxyphenol Methoxyphenyl Triazine enthaltenden Formulierung zugesetzt und die verfleckungsreduzierende Wirkung (Reduktion db) im Vergleich zu einer Formulierung ohne die erfindungsgemäßen Hilfsstoffe mittels einer *in vitro* Waschmethode bestimmt.

Als Beleg der verbesserten Auswaschbarkeit und verminderten Fleckenbildung der erfindungsgemäßen Zubereitungen wurden in vitro Untersuchungen durchgeführt, deren Ergebnisse in Tabelle 1 und 2 dargestellt sind.

Es wurden verschiedene Sonnenschutzemulsionen hinsichtlich der Bildung von gelben Flecken über einen in vitro Auftragungs-/ Waschzyklus untersucht. Es wurden dabei weiße vorgewaschene Baumwollmonitore (100% Baumwolle) verwendet. Dazu wurden je 50 mg der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät spectro-color (Dr. Lange); Farbmess-Software: spectral-QC, Version Messgeometrie: d/8°, Glanzkomponente ausgeschlossen, Lichtart: D65 (entsprechend mittlerem Tageslicht), Kalibrierstandard: LZM 268, Messöffnung: 10mm, Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen im Färbe- und Waschechtheitsgerät Linitest Plus (Atlas) (60°C, 1h, 20rpm, Ariel Compact Pulverwaschmittel, 10 Metallkugeln als Beiladung) und im Anschluss ein Spülvorgang (20°C, 15min, Leitungswasser).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät spectro-color (Dr. Lange).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, dass heißt, die Farben werden unabhängig von der Art ihrer Erzeugung und Wiedergabetechnik definiert.

Die entsprechende EU-Richtlinie ist DIN EN ISO 11664-4 "Farbmetrik- Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Tabelle 1: getestete Zubereitungen und deren Gelbwert-Reduktion von Flecken; db-Wert [%]**

| **INCI** | Bsp. 1 | Bsp. 2 | Bsp. 3 | Bsp. 4 | | Bsp. 5 | Bsp. 6 |
|---|---|---|---|---|---|---|---|
| Polyvinyl Alcohol (18-88) | | 1.00 | | | | 0.50 | 2.00 |
| Polyvinyl Alcohol (26-88) | | | 1.00 | | | | |
| Polyvinyl Alcohol (40-88) | | | | 1.00 | | | |
| Cetearyl Alcohol | 1.00 | 1.00 | 1.00 | 1.00 | | 1.00 | 1.00 |
| Myristyl Myristate | 1.00 | 1.00 | 1.00 | 1.00 | | 1.00 | 1.00 |
| Glyceryl Stearate SE | 1.00 | 1.00 | 1.00 | 1.00 | | 1.00 | 1.00 |
| Sodium Cetearyl Sulfate | 0.15 | 0.15 | 0.15 | 0.15 | | 0.15 | 0.15 |
| VP/Hexadecene Copolymer | 0.50 | 0.50 | 0.50 | 0.50 | | 0.50 | 0.50 |
| C18-38 Alkyl Hydroxystearoyl | | | | | | | |
| Stearate | 0.50 | 0.50 | 0.50 | 0.50 | | 0.50 | 0.50 |
| Silica Dimethyl Silylate | 0.50 | 0.50 | 0.50 | 0.50 | | 0.50 | 0.50 |
| Perfume | q.s. | q.s. | q.s. | q.s. | | q.s. | q.s. |
| Tocopheryl Acetate | 0.06 | 0.06 | 0.06 | 0.06 | | 0.06 | 0.06 |
| Panthenol | 1.05 | 1.05 | 1.05 | 1.05 | | 1.05 | 1.05 |
| Alcohol Denat. | 4.00 | 4.00 | 4.00 | 4.00 | | 4.00 | 4.00 |
| Trisodium EDTA | 0.20 | 0.20 | 0.20 | 0.20 | | 0.20 | 0.20 |
| Glycerin | 8.60 | 8.60 | 8.60 | 8.60 | | 8.60 | 8.60 |
| Sodium Hydroxide | q.s. | q.s. | q.s. | q.s. | | q.s. | q.s. |
| Phenoxyethanol | 0.50 | 0.50 | 0.50 | 0.50 | | 0.50 | 0.50 |
| Methylparaben | 0.30 | 0.30 | 0.30 | 0.30 | | 0.30 | 0.30 |
| Acrylates/C10-30 Alkyl Acrylate | | | | | | | |
| Crosspolymer | 0.10 | 0.10 | 0.10 | 0.10 | | 0.10 | 0.10 |
| Xanthan Gum | 0.40 | 0.40 | 0.40 | 0.40 | | 0.40 | 0.40 |
| Homosalate | 9.50 | 9.50 | 9.50 | 9.50 | | 9.50 | 9.50 |
| Ethylhexyl Salicylate | 4.75 | 4.75 | 4.75 | 4.75 | | 4.75 | 4.75 |
| Bis-Ethylhexyloxyphenol | | | | | | | |
| Methoxyphenyl Triazine | 3.50 | 3.50 | 3.50 | 3.50 | | 3.50 | 3.50 |
| Butyl Methoxydibenzoylmethane | 4.75 | 4.75 | 4.75 | 4.75 | | 4.75 | 4.75 |
| Phenylbenzimidazole Sulfonic Acid | 1.00 | 1.00 | 1.00 | 1.00 | | 1.00 | 1.00 |
| Octocrylene | 9.50 | 9.50 | 9.50 | 9.50 | | 9.50 | 9.50 |
| Aqua | ad 100 | ad 100 | ad 100 | ad 100 | | ad 100 | ad 100 |
| | | | | | | | |
| Gelbreduktion -db [%] vs Basis 1 | Basis 1 | -24 | -26 | -38 | | -11 | -50 |

**Tabelle 2: getestete Zubereitungen und deren Gelbwert-Reduktion von Flecken; db-Wert [%]**

| **INCI** | Bsp. 7 | Bsp. 8 | Bsp. 9 | Bsp. 10 |
|---|---|---|---|---|
| Polyvinyl Alcohol (18-88) | | 0.50 | 1.00 | 2.00 |
| C18-38 Alkyl Hydroxystearoyl Stearate | 1.00 | 1.00 | 1.00 | 1.00 |
| Silica Dimethyl Silylate | 1.00 | 1.00 | 1.00 | 1.00 |
| Glyceryl Stearate | 1.00 | 1.00 | 1.00 | 1.00 |
| Stearyl Alcohol | 1.25 | 1.25 | 1.25 | 1.25 |
| Sodium Stearoyl Glutamate | 0.25 | 0.25 | 0.25 | 0.25 |
| C12-15 Alkyl Benzoate | 2.00 | 2.00 | 2.00 | 2.00 |
| Butylene Glycol Dicaprylate/Dicaprate | 2.00 | 2.00 | 2.00 | 2.00 |
| Triacontanyl PVP | 1.00 | 1.00 | 1.00 | 1.00 |
| Perfume | q.s. | q.s. | q.s. | q.s. |
| Tocopheryl Acetate | 0.06 | 0.06 | 0.06 | 0.06 |
| Panthenol | 1.05 | 1.05 | 1.05 | 1.05 |
| Alcohol Denat. | 5.00 | 5.00 | 5.00 | 5.00 |
| Trisodium EDTA | 0.20 | 0.20 | 0.20 | 0.20 |
| Glycerin | 8.60 | 8.60 | 8.60 | 8.60 |
| Sodium Hydroxide | q.s. | q.s. | q.s. | q.s. |
| Acrylates/C10-30 Alkyl Acrylate Crosspolymer | 0.15 | 0.15 | 0.15 | 0.15 |
| Xanthan Gum | 0.40 | 0.40 | 0.40 | 0.40 |
| Hydroxyacetophenone | 0.40 | 0.40 | 0.40 | 0.40 |
| Ethylhexylglycerin | 0.15 | 0.15 | 0.15 | 0.15 |
| Tetrasodium Iminodisuccinate | 0.75 | 0.75 | 0.75 | 0.75 |
| Sodium Carboxymethylcellulose | 0.50 | 0.50 | 0.50 | 0.50 |
| Homosalate | 9.00 | 9.00 | 9.00 | 9.00 |
| Ethylhexyl Salicylate | 4.75 | 4.75 | 4.75 | 4.75 |
| Bis-Ethylhexyloxyphenol Methoxyphenyl Triazine | 4.00 | 4.00 | 4.00 | 4.00 |
| Butyl Methoxydibenzoylmethane | 4.75 | 4.75 | 4.75 | 4.75 |
| Phenylbenzimidazole Sulfonic Acid | 1.00 | 1.00 | 1.00 | 1.00 |
| Octocrylene | | | | |
| Ethylhexyl Triazone | 3.00 | 3.00 | 3.00 | 3.00 |
| Titanium Dioxide (nano) | 0.82 | 0.82 | 0.82 | 0.82 |
| Silica | 0.15 | 0.15 | 0.15 | 0.15 |
| Dimethicone | 0.04 | 0.04 | 0.04 | 0.04 |
| Aqua | ad 100 | ad 100 | ad 100 | ad 100 |
| | | | | |
| Gelbreduktion -db [%] vs Basis 2 | Basis 2 | -18 | -22 | -47 |

**Fazit:** Es zeigte sich, dass durch den erfindungsgemäßen Zusatz von Polyvinylalkohol sich die UV-Filter 4-(tert.-Butyl)-4'-methoxydibenzoylmethan (INCI Butyl Methoxydibenzoylmethane) und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) leichter und vollständiger aus dem Baumwollträger auswaschen lassen und die durch diese UV-Filter hervorgerufene Textilverfleckung signifikant reduziert wird. Ebenfalls ist eine Konzentrations-Wirkungs-Beziehung zu erkennen.

### Beispiele für Rezepturen/Sonnenschutzmittel mit denen das erfindungsgemäße Verfahren und die erfindungsgemäße Verwendung durchgeführt werden können:

Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | Bsp. 11 | Bsp. 12 | Bsp. 13 | Bsp. 14 | Bsp. 15 | Bsp. 16 | Bsp. 17 | Bsp. 18 | Bsp. 19 | Bsp. 20 |
|---|---|---|---|---|---|---|---|---|---|---|
| Triacontanyl PVP | | 0,50 | 1,00 | 1,00 | 1,00 | 1,00 | 1,00 | 1,00 | 1,00 | 1,00 |
| Hydroxyacetophenone | 0,40 | 0,40 | | | | | 0,40 | | | |
| Panthenol | | 1,00 | 1,00 | | | | | | | |
| Glyceryl Stearate Citrate | 2,00 | 2,00 | | | | | | | | |
| Glyceryl Stearate | | | 1,00 | 1,00 | | 1,00 | 1,00 | | | 1,00 |
| Sodium Stearoyl Glutamate | | | 0,30 | 0,30 | 0,00 | 0,30 | 0,25 | | | 0,40 |
| Glyceryl Stearate SE | | | | | 1,00 | | | | | |
| Ethylhexylglycerin | 0,30 | 0,30 | | 0,30 | | | 0,15 | | | |
| Piroctone Olamine | | | 0,10 | 0,10 | | 0,10 | | | | |
| C12-15 Alkyl Benzoate | 4,50 | 4,50 | 2,00 | 2,00 | | 2,00 | 2,00 | | | 2,00 |
| Myristyl Myristate | | 0,50 | | | 1,00 | | | | | |
| Hydrogenated Coco-Glycerides | 1,00 | 1,00 | | | | | | | | |
| Butylene Glycol Dicaprylate/Dicaprate | 4,50 | | 2,00 | 2,00 | | 2,00 | 2,00 | 2,00 | 2,00 | 2,00 |
| Dicaprylyl Carbonate | 2,00 | | | | | | | | | |
| C18-38 Alkyl Hydroxystearoyl Stearate | | | 1,00 | 1,00 | 0,50 | 1,00 | 1,00 | | | |
| Copernicia Cerifera Cera | | | | | | | | | 0,50 | |
| C18-36 Acid Triglyceride | | | | | | | | 1,00 | | 1,00 |
| Acrylates/C10-30 Alkyl Acrylate Crosspolymer | 0,15 | 0,15 | 0,15 | 0,15 | 0,10 | 0,15 | 0,15 | 0,35 | 0,45 | 0,10 |
| Xanthan Gum | 0,30 | 0,30 | 0,40 | 0,40 | 0,40 | 0,40 | 0,40 | | | 0,12 |
| Cetyl Alcohol | 0,50 | | | | | | | | | |
| Microcrystalline Cellulose | | | | | | | | | 1,00 | 1,00 |
| Sodium Cetearyl Sulfate | | | | | 0,15 | | | | | |
| Ceteareth-20 | | | | | | | | 1,50 | 1,50 | |
| Silica Dimethyl Silylate | | | 1,00 | 1,00 | 0,50 | 1,00 | 1,00 | | | |
| VP/Hexadecene Copolymer | 0,50 | | | | | | | | | |
| Parfum | q.s. | q.s. | q.s. | q.s. | q.s. | q.s. | q.s. | q.s. | q.s. | q.s. |
| Glycerin | 8,00 | 8,00 | 7,50 | 7,50 | 8,00 | 7,50 | 7,50 | 5,00 | 5,00 | 5,00 |
| Citric Acid | q.s. | q.s. | q.s. | q.s. | q.s. | q.s. | q.s. | q.s. | q.s. | q.s. |
| Sodium Hydroxide | q.s. | q.s. | q.s. | q.s. | q.s. | q.s. | q.s. | q.s. | q.s. | q.s. |
| Phenoxyethanol | | | 0,50 | 0,50 | 0,50 | 0,50 | | 0,50 | 0,50 | 0,50 |
| Methylparaben | | | | | 0,30 | | | 0,30 | 0,30 | 0,30 |
| Ethylparaben | | | | | | | | 0,20 | 0,20 | 0,20 |
| Stearyl Alcohol | | 0,25 | 1,00 | 1,00 | 1,00 | 1,00 | 1,25 | | | |
| Polyvinyl Alcohol (18-88) | | 1,00 | | | | | | | | |
| Polyvinyl Alcohol (26-88) | | | | | 0,75 | | | | | |
| Polyvinyl Alcohol (40-88) | 0,50 | | 0,50 | | | 0,50 | | 0,50 | | 0,50 |
| Polyvinyl Alcohol (8-88) | | | | 1,00 | | | 1,00 | | 1,00 | |
| | | | | | | | | | | |
| Alcohol Denat. | 5,00 | 5,00 | 3,00 | 3,00 | 4,00 | 3,00 | 5,00 | 4,00 | 4,00 | 6,00 |
| Trisodium EDTA | 0,20 | 0,20 | 0,20 | 0,20 | 0,20 | 0,20 | 0,20 | 0,20 | 0,20 | 0,20 |
| Homosalate | 0,00 | 9,00 | 9,00 | 9,00 | 9,50 | 9,00 | 9,00 | 9,00 | 9,00 | 9,00 |
| Octocrylene | 9,00 | 9,00 | | | 9,50 | | | 8,00 | 8,00 | |
| Ethylhexyl Salicylate | 0,00 | 4,50 | 4,75 | 4,75 | 4,75 | 4,75 | 4,75 | 4,75 | 4,75 | 4,75 |
| Butyl Methoxydibenzoylmethane | 4,50 | 4,50 | 4,75 | 4,75 | 4,75 | 4,75 | 4,75 | 4,50 | 4,50 | 4,75 |
| Titanium Dioxide | 6,00 | 3,00 | | | | | 1,00 | | | |
| Bis-Ethylhexyloxyphenol Methoxyphenyl Triazine | 3,00 | | 4,00 | 4,00 | 3,50 | 4,00 | 4,00 | 3,50 | 3,00 | 4,00 |
| Ethylhexyl Triazone | | | 3,00 | 3,00 | | 3,00 | 3,00 | | | 3,00 |
| Phenylbenzimidazole Sulfonic Acid | | | 1,00 | 1,00 | 1,00 | 1,00 | 1,00 | 1,50 | 1,50 | 1,00 |
| Aqua | ad 100 | ad 100 | ad 100 | ad 100 | ad 100 | ad 100 | ad 100 | ad 100 | ad 100 | ad 100 |

## Patentansprüche

1. Verfahren zur Erleichterung der Auswaschbarkeit von UV-Lichtschutzfilter enthaltenden kosmetischen Zubereitungen aus Textilien, **dadurch gekennzeichnet, dass** dem Kosmetikum Polyvinylalkohol zugesetzt wird.

2. Verfahren zur Reduzierung der durch UV-Lichtschutzfilter enthaltenden kosmetischen Zubereitungen hervorgerufenen Textilverfleckung, **dadurch gekennzeichnet, dass** dem Kosmetikum Polyvinylalkohol zugesetzt wird.

3. Verwendung von Polyvinylalkohol in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

4. Verwendung von Polyvinylalkohol in UV-Lichtschutzfilter enthaltenden kosmetischen Zubereitungen zur Reduzierung der durch die Zubereitung hervorgerufene Textilverfleckung.

5. Verfahren oder Verwendung nach einem der vorhergehenden Ansprüche, **dadurch gekennzeichnet, dass** die kosmetische Zubereitung bzw. das Kosmetikum Verbindungen 4-(tert.-Butyl)-4'-methoxydibenzoylmethan (INCI Butyl Methoxydibenzoylmethane) und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

6. Verfahren oder Verwendung nach einem der vorhergehenden Ansprüche, **dadurch gekennzeichnet, dass** die kosmetische Zubereitung bzw. das Kosmetikum von 0,01 bis 2,5 Gewichts-% Polyvinylalkohol, bezogen auf das Gesamtgewicht der Zubereitung, enthält.

7. Verfahren oder Verwendung nach einem der vorhergehenden Ansprüche, **dadurch gekennzeichnet, dass** die Zubereitung eine oder mehrere Komplexbildner gewählt aus der Gruppe
- 1-Hydroxyethan-(1,1-diphosphonsäure)/ HEDP
- Aminotrimethylenphosphonsäure/ ATMP
- Diethylentriaminpenta(methylenphosphonsäure)/ DTPMP
- Ethylendiamintetra(methylenphosphonsäure/ EDTMP
- Phosphonobutan-tricarbonsäure/ PBTC
- Iminodisuccinat (IDS)
- Natriumpolyphosphat
- Tetranatriumpyrophosphat
- Hydroxamsäure
- Polygalacturonsäure
- Bernsteinsäure
- Ameisensäure
- Äpfelsäure
- Ethylendiamintetraessigsäure (EDTA)
und/oder deren Alkalisalze enthalten.

8. Verfahren oder Verwendung nach einem der vorhergehenden Ansprüche, **dadurch gekennzeichnet, dass** das Kosmetikum 1-Hydroxy-4-methyl-6-(2,4,4-trimethylpentyl)-2(1H)-pyridon (Pirocton) und/oder dessen Monoethanolamin-Salz enthält.

9. Verfahren oder Verwendung nach einem der vorhergehenden Ansprüche, **dadurch gekennzeichnet, dass** man dem Kosmetikum Siloxanelastomere zusetzt.

10. Verfahren oder Verwendung nach einem der vorhergehenden Ansprüche, **dadurch gekennzeichnet, dass** man dem Kosmetikum Polysaccharide zusetzt.

11. Verfahren oder Verwendung nach einem der vorhergehenden Ansprüche, **dadurch gekennzeichnet**, das man dem Kosmetikum 4-Hydroxyacetophenon zusetzt.

12. Verfahren oder Verwendung nach einem der vorhergehenden Ansprüche, **dadurch gekennzeichnet, dass** als Polyvinylalkohol einen Hydrolysegrad (Verseifungsgrad) von 86 bis 89 Mol-% aufweist.

13. Verfahren oder Verwendung nach einem der vorhergehenden Ansprüche, **dadurch gekennzeichnet, dass** als Polyvinylalkohol eine Viskosität gemessen nach DIN 53015 (4%ige wässrige Lösung bei 20 °C) von 3 bis 50 mPas aufweist.

## Claims

1. Process for facilitating the ability of cosmetic preparations comprising UV photoprotective filters to be washed out of textiles, **characterized in that** polyvinyl alcohol is added to the cosmetic.

2. Process for reducing the textile staining caused by cosmetic preparations comprising UV photoprotective filters, **characterized in that** polyvinyl alcohol is added to the cosmetic.

3. Use of polyvinyl alcohol in cosmetic preparations comprising UV photoprotective filters for facilitating the ability of UV photoprotective filters to be washed out of textiles contaminated with the preparations.

4. Use of polyvinyl alcohol in cosmetic preparations comprising UV photoprotective filters for reducing the textile staining caused by the preparation.

5. Process or use according to any of the preceding claims, **characterized in that** the cosmetic preparation or the cosmetic comprises compounds 4-(tert-butyl)-4'-methoxydibenzoylmethane (INCI Butyl Methoxydibenzoylmethane) and/or 2,4-bis{[4-(2-ethylhexyloxy)-2-hydroxy]phenyl}-6-(4-methoxyphenyl)-1,3,5-triazine (INCI Bis-Ethylhexyloxyphenol Methoxyphenyl Triazine).

6. Process or use according to any of the preceding claims, **characterized in that** the cosmetic preparation or the cosmetic comprises from 0.01 to 2.5% by weight polyvinyl alcohol, based on the total weight of the preparation.

7. Process or use according to any of the preceding claims, **characterized in that** the preparation comprises one or more chelating agents selected from the group comprising
- 1-hydroxyethane-(1,1-diphosphonic acid)/ HEDP
- aminotrimethylenephosphonic acid/ ATMP
- diethylenetriaminepenta(methylenephosphonic acid)/ DTPMP
- ethylenediaminetetra(methylenephosphonic acid)/ EDTMP
- phosphonobutanetricarboxylic acid/ PBTC
- iminodisuccinate (IDS)
- sodium polyphosphate
- tetrasodium pyrophosphate
- hydroxamic acid
- polygalacturonic acid
- succinic acid
- formic acid
- malic acid
- ethylenediaminetetraacetic acid (EDTA)
and/or alkali metal salts thereof.

8. Process or use according to any of the preceding claims, **characterized in that** the cosmetic comprises 1-hydroxy-4-methyl-6-(2,4,4-trimethylpentyl)-2(1H)-pyridone (piroctone) and/or the monoethanolamine salt thereof.

9. Process or use according to any of the preceding claims, **characterized in that** siloxane elastomers are added to the cosmetic.

10. Process or use according to any of the preceding claims, **characterized in that** polysaccharides are added to the cosmetic.

11. Process or use according to any of the preceding claims, **characterized in that** 4-hydroxyacetophenone is added to the cosmetic.

12. Process or use according to any of the preceding claims, **characterized in that** the polyvinyl alcohol has a degree of hydrolysis (degree of saponification) of 86 to 89 mol%.

13. Process or use according to any of the preceding claims, **characterized in that** the polyvinyl alcohol has a viscosity, measured in accordance with DIN 53015 (4% aqueous solution at 20°C), of 3 to 50 mPas.

## Revendications

1. Procédé visant à faciliter la rinçabilité de préparations cosmétiques contenant des filtres de protection contre la lumière UV à partir de textiles, **caractérisé en ce que** de l'alcool polyvinylique est ajouté au produit cosmétique.

2. Procédé visant à réduire les taches sur des textiles causées par des préparations cosmétiques contenant des filtres de protection contre la lumière UV, **caractérisé en ce que** de l'alcool polyvinylique est ajouté au produit cosmétique.

3. Utilisation d'alcool polyvinylique dans des préparations cosmétiques contenant des filtres de protection contre la lumière UV pour faciliter la rinçabilité des filtres de protection contre la lumière UV à partir de textiles contaminés avec les préparations.

4. Utilisation d'alcool polyvinylique dans des préparations cosmétiques contenant des filtres de protection contre la lumière UV pour réduire les taches sur des textiles causées par la préparation.

5. Procédé ou utilisation selon l'une quelconque des revendications précédentes, **caractérisé en ce que** la préparation cosmétique ou le produit cosmétique contient les composés 4-(tert.-butyl)-4'-méthoxydibenzoylméthane (INCI : Butyl Methoxydibenzoylmethane) et/ou 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

6. Procédé ou utilisation selon l'une quelconque des revendications précédentes, **caractérisé en ce que** la préparation cosmétique ou le produit cosmétique contient de 0,01 à 2,5 % en poids d'alcool polyvinylique, par rapport au poids total de la préparation.

7. Procédé ou utilisation selon l'une quelconque des revendications précédentes, **caractérisé en ce que** la préparation contient un ou plusieurs complexants choisis dans le groupe constitué par :
- l'acide 1-hydroxyéthane-(1,1-diphosphonique)/HEDP,
- l'acide aminotriméthylène-phosphonique/ATMP,
- l'acide diéthylène-triamine-penta(méthylène-phosphonique)/DTPMP,
- l'acide éthylène-diamine-tétra(méthylène-phosphonique)/EDTMP,
- l'acide phosphonobutane-tricarboxylique/PBTC,
- l'iminodisuccinate (IDS),
- le polyphosphate de sodium,
- le pyrophosphate de tétrasodium,
- l'acide hydroxamique,
- l'acide polygalacturonique,
- l'acide succinique,
- l'acide formique,
- l'acide malique,
- l'acide éthylène-diamine-tétraacétique (EDTA),
et/ou leurs sels alcalins.

8. Procédé ou utilisation selon l'une quelconque des revendications précédentes, **caractérisé en ce que** le produit cosmétique contient de la 1-hydroxy-4-méthyl-6-(2,4,4-triméthylpentyl)-2(1H)-pyridone (piroctone) et/ou son sel de monoéthanolamine.

9. Procédé ou utilisation selon l'une quelconque des revendications précédentes, **caractérisé en ce que** des élastomères de siloxane sont ajoutés au produit cosmétique.

10. Procédé ou utilisation selon l'une quelconque des revendications précédentes, **caractérisé en ce que** des polysaccharides sont ajoutés au produit cosmétique.

11. Procédé ou utilisation selon l'une quelconque des revendications précédentes, **caractérisé en ce que** de la 4-hydroxyacétophénone est ajoutée au produit cosmétique.

12. Procédé ou utilisation selon l'une quelconque des revendications précédentes, **caractérisé en ce que** l'alcool polyvinylique présente un degré d'hydrolyse (degré de saponification) de 86 à 89 % en moles.

13. Procédé ou utilisation selon l'une quelconque des revendications précédentes, **caractérisé en ce que** l'alcool polyvinylique présente une viscosité mesurée selon DIN 53015 (solution aqueuse à 4 % à 20 °C) de 3 à 50 mPas.
